# DEMANDE DE BREVET EUROPEEN

(11) **EP 2 644 142 A1**
(43) Date de publication de la demande: **02.10.2013**
(21) Numéro de dépôt: 13160932.3
(22) Date de dépôt: 25.03.2013
(51) Int. Cl.: A61B 17/72, A61B 17/74

(54) **Implant pour les fractures proximales d'os longs**

(30) Priorité: 28.03.2012 CH 4362012; 24.05.2012 CH 7252012
(71) Demandeur: Chirmat Sàrl, 1870 Monthey (CH); Université de Savoie, 73011 Chambery Cedex (FR); Hepia, 1202 Genève (CH); Tural, 74970 Marignier (FR)
(72) Inventeur: Bonjour, Christian, 1268 Begnins (CH); Arlettaz, Yvan, 1870 Monthey (CH); Vacher, Pierre, 74570 Thorens-Glières (FR); Toussaint, Franck, 74000 Annecy (FR); Billard, Rémi, 83700 Saint-Raphaël (FR); Devun-Daulon, Laure, 74490 Saint-Jérôme (FR); Gradel, Thomas, 74970 Marignier (FR); Vittecoq, Eric, 74570 Groisy (FR)
(74) Mandataire: P&TS SA (AG, Ltd.)

(57) **Abrégé**

Implant chirurgical pour le traitement des fractures d'os longs, notamment du fémur, avec une section intramédullaire (52) de forme allongée, adaptée pour l'insertion dans le canal médullaire (37), une section extramédullaire (56) sensiblement parallèle à la section intramédullaire (52), une anse courbe (57) déformable reliant la section intramédullaire (52) à la section extramédullaire (56), une vis inclinée (61) traversant ladite section intramédullaire (52) et ladite section extramédullaire (56). Par la déformation de l'anse (57) on peut obtenir un réglage continu de l'angle (α) de la vis céphalique (61) et une adaptation optimale de l'implant à l'anatomie individuelle. De par sa géométrie, cet implant induit une protection du massif trochantérien et limite les sollicitations mécaniques entre la vis céphalique (61) et le reste de l'implant.

## Description

### Domaine technique

La présente invention concerne un dispositif implantable pour le traitement des fractures proximales d'os longs. Notamment, mais pas exclusivement, elle concerne un implant utilisable pour le traitement des fractures du massif trochantérien fémoral. La présente invention s'adresse également aux fractures de l'humérus proximal ou d'autres os longs comportant des problématiques et des thérapies similaires.

### Etat de la technique

La technique présentée ci-dessous concerne les fractures du fémur proximal ; les techniques pour le traitement des fractures de tous les os longs sont similaires.

Les fractures du massif trochantérien fémoral sont des fractures concernant la partie proximale du fémur près de l'articulation de la hanche. Ces traumatismes font partie des fractures appelées dans le langage courant des fracture du col du fémur et touchent le plus souvent des personnes âgées. Leur thérapie se base, dans la majorité des cas, sur un traitement chirurgical rapide avec une réduction anatomiquement correcte suivie de la pose d'un implant qui stabilise la fracture. Il a été démontré que ces procédures permettent une rééducation à la marche dans des temps assez rapides et améliorent considérablement le taux de guérison.

Les techniques actuellement employées comportent la stabilisation de la fracture soit par des implants endomédullaires insérés dans le canal diaphysaire, soit par des plaques ou des lames vissées sur l'os. Dans tous les cas, on utilise une ou des vis dites céphaliques ou autres dispositifs orientés selon l'axe du col fémoral pour stabiliser la tête du fémur.

Le dispositif divulgué par EP0546460B1, par exemple, comprend une plaque de stabilisation du trochanter connectable par une vis à un clou centromédullaire et comprenant deux vis inclinées, de part et d'autre du canal médullaire pour la stabilisation de la tête et du col du fémur. US7632272, d'autre part, divulgue une fixation intramédullaire avec une vis inclinée destinée à s'enfoncer dans la tête du fémur et une plaquette pour la stabilisation du grand trochanter.

L'angulation du col fémoral par rapport à la diaphyse fémorale présente une forte variabilité par rapport à la morphologie individuelle, au sexe et à l'âge. Il est donc nécessaire de disposer d'implants reconstruisant l'angulation anatomiquement correcte pour chaque patient. Cela conduit actuellement à une multiplication du nombre d'implants à fournir à chaque hôpital pour permettre le traitement en urgence de ce type de fracture.

Par ailleurs, les fractures dites intertrochantériennes ou pertrochantériennes provoquent souvent une rupture de la partie haute du fémur appelée 'grand trochanter' dans laquelle s'attachent plusieurs muscles responsables de la stabilité et de la marche et qui y exercent des forces importantes. Les implants généralement utilisés ne permettent pas toujours une stabilisation adéquate des fractures du grand trochanter.

### Bref résumé de l'invention

Un but de la présente invention est de proposer un implant exempt des limitations des dispositifs connus, et notamment facilement adaptable à une pluralité de configurations anatomiques, de façon à réduire le nombre de référence d'implants dans les hôpitaux.

Un autre but de l'invention est de proposer un implant permettant, de par sa géométrie, une protection efficace du grand trochanter dans les fractures intertrochantériennes ou pertrochantériennes et une limitation des sollicitations mécaniques entre la vis céphalique 61 et le reste de l'implant.

Selon l'invention, ces buts sont atteints au moyen de l'objet des revendications annexées, et notamment par un implant chirurgical avec une section intramédullaire de forme allongée, adaptée pour l'insertion dans le canal médullaire d'un os long fracturé, une section extramédullaire sensiblement parallèle à la section intramédullaire, une anse courbe déformable pour s'adapter à l'anatomie du patient reliant la section intramédullaire à la section extramédullaire, une vis céphalique inclinée traversant ladite section intramédullaire et ladite section extramédullaire.

### Brève description des figures

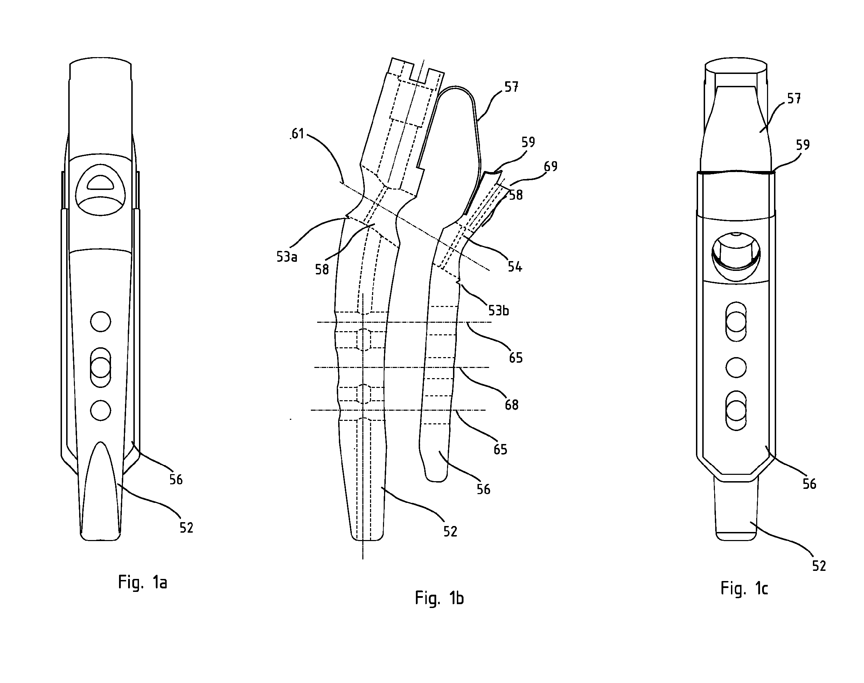

Un exemple de réalisation d'un tel implant est illustré par les Fig. 1a-1c.

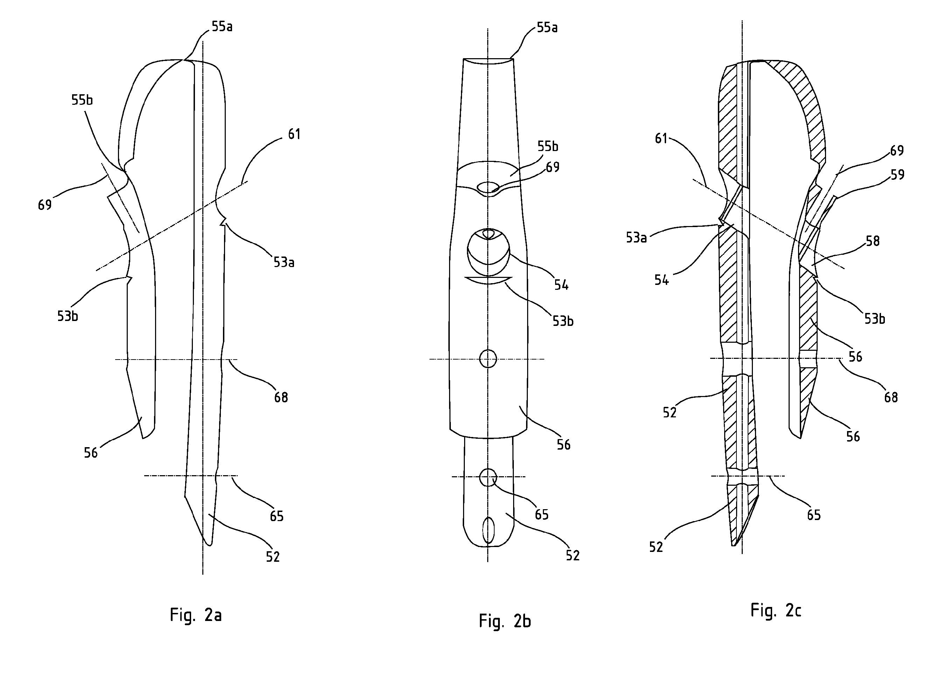

D'autres variantes de réalisation de l'invention sont indiquées dans la description illustrée par les Fig. 2a-2c.

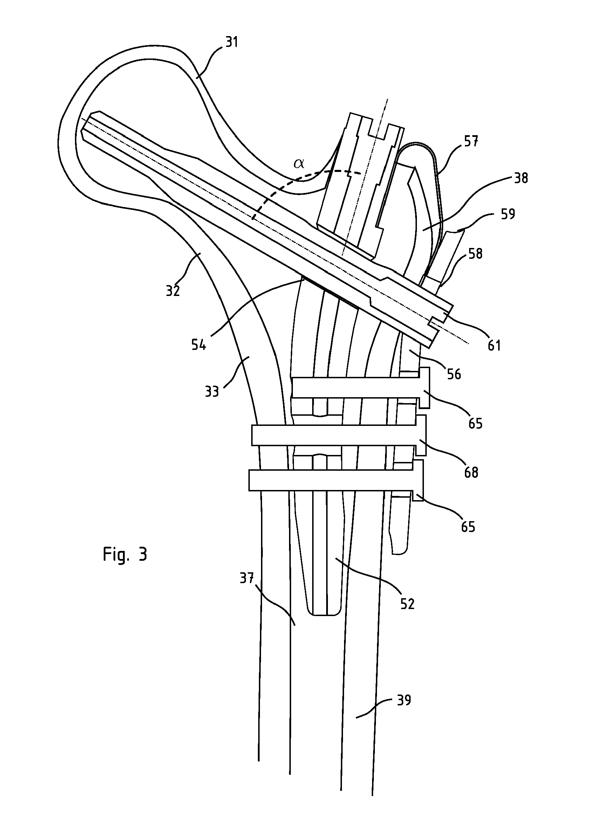

La Fig. 3 montre, de façon simplifiée, un implant selon un aspect de l'invention en position dans la partie proximale d'un fémur, en section frontale.

Les caractéristiques identiques présentes en plusieurs figures sont indiquées par le même signe de référence. Le même signe de référence est utilisé en quelques cas pour indiquer les vis de verrouillage et fixation, ou leur axe dans une représentation simplifiée.

### Exemple(s) de mode de réalisation de l'invention

Les modes de réalisation présentés font référence uniquement à un implant pour les fractures du fémur humain. La présente invention toutefois comprend aussi des variantes spécifiques pour le traitement de fractures d'autres os longs, notamment de l'humérus. Cette invention peut aussi s'appliquer dans le domaine vétérinaire.

Les Fig. 1a-1c illustrent un élément de l'implant selon un aspect de l'invention et qui comporte une section intramédullaire 52, de forme allongée (droite ou incurvée) et adaptée pour l'insertion dans le canal médullaire 37 (visible sur la Fig. 3) de l'os concerné, par exemple du fémur. L'implant comporte également une section extramédullaire 56, essentiellement parallèle à la section intramédullaire 52.

L'anse courbe 57 reliant la section intramédullaire 52 à la section extramédullaire 56 a une forme adaptée pour épouser le profil anatomique du grand trochanter. Avantageusement, l'anse 57 est déformable pour que l'implant s'adapte à la morphologie individuelle de chaque patient et faciliter l'insertion de l'implant dans l'os.

La déformation de l'anse 57 peut être obtenue de plusieurs façons. Dans une variante, représentée schématiquement sur la Fig. 1, l'anse 57 est constituée par une lame déformable élastiquement ou non, de par son épaisseur relativement réduite. Selon une autre variante, représentée par les Fig. 2a-2c, l'anse 57 comporte un certain nombre de segments de section réduite 55a-55d formant des charnières déformables. Grâce à cette caractéristique le chirurgien peut déformer l'implant et obtenir le degré de courbure souhaité par application d'une force raisonnable.

La Fig. 3 montre schématiquement l'implant de l'invention en place. On peut voir la section intramédullaire 52 insérée dans le canal diaphysaire 37, d'un fémur 39, tandis que la section extramédullaire 56 s'appuie sur une face latérale de l'os, et l'anse 57 entoure et protège le grand trochanter 38. Le canal diaphysaire 37 aura préalablement été ouvert et alésé au diamètre souhaité par des techniques connues.

L'implant de l'invention comporte aussi une vis céphalique inclinée 61 traversant la section intramédullaire 52 et la section extramédullaire 56 par deux ouvertures 54, respectivement 58. L'angle α entre la vis 61 et la section intramédullaire 52 correspond à l'inclinaison du col du fémur, de façon à ce que la vis 61 puisse traverser le col 32 selon son axe, pénétrer dans la tête du fémur 31 et la rendre solidaire.

De façon importante, l'angle α de la vis 61 peut être modifié en agissant sur la courbure de l'anse 57, et l'alignement des ouvertures 58, 54, afin de s'adapter au mieux à l'anatomie de chaque patient. L'angle α idéal peut être déterminé par des radiographies préopératoires de l'anche saine controlatérale, ou préalablement à l'implant, lorsque la fracture est réduite, grâce à un appareil de radioscopie chirurgicale. L'implant est préréglé avant introduction dans le fémur, et un éventuel réajustement une fois l'implant en place peut être réalisé en appuyant sur l'excroissance 59. Deux encoches 53a et 53b permettent de matérialiser par radioscopie l'axe de la vis céphalique 61 avant introduction de celle-ci, afin de déterminer si un réajustement est nécessaire ou pas. La forme des ouvertures 54 et 58 est adaptée à ce mouvement relatif entre les parties intramédullaire 52 et extramédullaire 56.

La vis céphalique 61, après positionnement, est préférablement bloquée en rotation, par rapport à la partie intramédullaire 52, et/ou par rapport à la partie extramédullaire 56 comme le montre la figure 2c, par la vis 69. Les sollicitations mécaniques générées par la vis céphalique sur le reste de l'implant sont ainsi réduites par rapport aux implants de type conventionnel.

Plusieurs moyens de fixation peuvent être envisagés pour maintenir en place les parties intra- et extramédullaires de l'implant.

Pour la partie intramédullaire, une ou plusieurs vis de verrouillage 65 sont vissées dans l'os cortical. Pour la partie extramédullaire, une ou plusieurs vis 68 sont soit vissées dans l'os cortical, soit dans la partie intramédullaire de l'implant.

Les trous de passage des vis 61, 65, 68 dans les parties intra- et extramédullaires 56, 52 peuvent être circulaires ou oblong. Préférablement la section intramédullaire 52, la section extramédullaire 56 et l'anse 57 sont constituées par un seul élément solide, par exemple un élément métallique en titane ou en alliage biocompatible, ou tout autre matériau biocompatible. L'implant de l'invention comporte ainsi un nombre très réduit de pièces détachables. Cet implant monobloc peut toutefois être fabriqué en plusieurs parties assemblées (par soudage par exemple).

### Numéros de référence employés sur les figures

| | |
|---|---|
| 31 | tête du fémur |
| 32 | col du fémur |
| 33 | fracture |
| 37 | canal diaphysaire |
| 38 | grand trochanter |
| 39 | diaphyse |
| 52 | section intramédullaire |
| 53 | encoche de contrôle angulation de la vis céphalique |
| 54 | guidage intramédullaire vis céphalique |
| 55a-d | charnière |
| 56 | section extramédullaire |
| 57 | anse |
| 58 | guidage extramédullaire vis céphalique |
| 59 | zone d'appui du réglage angulaire |
| 61 | vis céphalique |
| 65 | vis de verrouillage partie intramédullaire |
| 68 | vis de verrouillage partie extramédullaire |
| 69 | vis de blocage en rotation de la vis céphalique |
| α | angle |

## Revendications

1. Implant chirurgical pour le traitement des fractures d'os longs, avec une section intramédullaire (52) de forme allongée, adaptée pour l'insertion dans le canal médullaire (37) d'un os long fracturé, une section extramédullaire (56) sensiblement parallèle à la section intramédullaire (52), une anse courbe (57) reliant la section intramédullaire (52) à la section extramédullaire (56), une vis inclinée (61) traversant ladite section intramédullaire (52) et ladite section extramédullaire (56), **caractérisée en ce que** ladite anse (57) est déformable pour s'adapter à l'anatomie du patient.

2. L'implant de la revendication précédente dans lequel ladite anse courbe est adaptée pour entourer et protéger le massif trochantérien lorsque l'implant est en place.

3. L'implant de la revendication 1, dans lequel ladite anse (57) comporte une lame déformable élastiquement ou non par application d'une force avant ou durant l'implantation.

4. L'implant d'une des revendications précédentes comprenant de moyens (69) pour bloquer la rotation de la dite vis inclinée (61).

5. L'implant d'une des revendications précédentes, dans lequel la dite section intramédullaire (52), ladite section extramédullaire (56) et ladite anse (57) sont constituées par un assemblage de plusieurs éléments.

6. L'implant d'une des revendications précédentes, dans lequel la dite section intramédullaire (52), ladite section extramédullaire (56) et ladite anse (57) sont constituées par un seul élément monobloc.

7. L'implant d'une des revendications précédentes, dans lequel la dite section extramédullaire (56) comporte une ou plusieurs ouvertures (59) permettant sa fixation sur une face dudit os par des vis (68) ou tout autre moyen de fixation.

8. L'implant de la revendication précédente, dans lequel ladite anse présente des segments de section réduite (55a-55d) permettant sa déformation par application d'une force.
